Europäisches Patentamt

European Patent Office

Office européen des brevets

(11) Publication number: **0 480 458 A2**

## EUROPEAN PATENT APPLICATION

(21) Application number: **91117380.5**

(22) Date of filing: **11.10.91**

(51) Int. Cl.5: **A61K 31/40**

(30) Priority: **12.10.90 IT 2172290**

(43) Date of publication of application:
**15.04.92 Bulletin 92/16**

(84) Designated Contracting States:
**AT BE CH DE DK ES FR GB GR IT LI LU NL SE**

(71) Applicant: **ZAMBON GROUP S.p.A.**
**Via della Chimica, 9**
**I-36100 Vicenza(IT)**

(72) Inventor: **Della Bella, Davide**
**Piazza Leonardo da Vinci, 3**
**I-20133 Milano(IT)**
Inventor: **Carenzi, Angelo**
**Via Rossini, 9**
**I-21052 Busto Arsizio (VA)(IT)**
Inventor: **Cibin, Mauro**
**Via Lovarini, 6**
**I-35100 Padova(IT)**
Inventor: **Gentile, Nicola**
**Via Barroccio dal Borgo, 8**
**I-35100 Padova(IT)**

(54) **Process for the preparation of pharmaceutical compositions containing Viminol useful in the treatment of drug dependences.**

(57) Viminol, 1-(2-chlorobenzyl)-2-[(1-hydroxy-2-di-sec.butylamino)]-ethylpyrrole, is a known drug with analgesic activity.

Now it has been found that it may be useful also in the therapeutical treatment of drug dependences.

EP 0 480 458 A2

The present invention relates to a process for the preparation of pharmaceutical compositions useful in the treatment of drug dependences containing Viminol as active ingredient.

Viminol (Merck Index, XI Ed., No. 9885, pages 1569-70) is the common name of the compound 1-(2-chlorobenzyl)-2-[(1-hydroxy-2-di-sec.butylamino)]-ethylpyrrole.

The compound has three asymmetric carbon atoms but it exists only in the form of 6 stereoisomers because of the presence of two equivalent sec.butyl groups on the amino nitrogen.

Viminol is a strong analgesic drug used in the treatment of pain of different origin and it is commercialized as a mixture of stereoisomers with the registered trademark Dividol® (Zambon Group S.p.A.) in the form of 4-hydroxybenzoic acid salt.

Nowadays, the treatment of drug dependences is carried out mainly by administration to the patient of increasing doses of drugs having an antagonistic activity to opiate receptors such as Naltrexone (Merck Index, XI Ed., No. 6278, page 1066) or of drugs having an agonistic activity but a low capacity to induce dependence such as Metadone (Merck Index, XI Ed., No. 5852, page 936).

However, this last drug is not completely free of inducing dependence.

Experimental studies on Viminol have demonstrated that the compound has only a low affinity to opiate receptors [Della Bella D. et al., Pharmacological Research Communications, vol. 8, No. 2, pages (1976), 111-126].

We have now found that Viminol does not induce dependence and, surprisingly, exerts a beneficial action in reducing the abstinence phenomena caused by the suspension of the administration of morphine and morphine-like compounds; consequently Viminol can be useful in the treatment of drug dependence.

Thanks to these characteristics, Viminol can also help the beginning of a therapy with Naltrexone.

Therefore, object of the present invention is a process for the preparation of pharmaceutical compositions useful in the treatment of drug dependence, containing Viminol.

Such compositions can be in the form of different common pharmaceutical preparations such as tablets, capsules, coated tablets, suppositories, drinking solutions, injectable solutions and freezedried powders.

They contain a therapeutically effective amount of Viminol together with pharmaceutical acceptable excipients.

The efficacy of Viminol in the treatment of drug dependence has been demonstrated in mouse by modifying the jumping test described by Way E.L. et al. in J. Pharmacol. Exp. Ther., 167, (1969), 1-8.

The modified method and the test results are illustrated in details in example 1.

The drug dependences which can be treated with Viminol are those caused by an excessive use of morphine, heroin and morphine-like compounds in general.

For the practical use, a daily dose ranging from 100 to 800 mg will be administered to the patient.

The therapeutic dose will be ensured by the daily administration of one or more compositions whose preparation is object of the invention and it will be adapted to the patient depending on some factors such as the individual answer, the administration route and the selected kind of composition.

In order to better illustrate the present invention, the following example is now given.

Example

The method used for carrying out the jumping test was modified as follows: an osmotic micropump "Alzet 2002"® (registered trademark) of Alza Corporation) containing a 155 mM solution of morphine hydrochloride was grafted on to the skin of male mice weighing about 22-24 g. The technical characteristics of the pump (reservoir = 100 $\mu$l) allow a prolonged release of 1 $\mu$l/hour for 72 hours.

During the release of morphine, the animals were treated orally twice a day for three days with Dividol® at the dose of 50 mg/kg. After about 75 hours from the grafting, Naloxone (Merck Index, XI Ed., No. 6277, page 1006) (10 mg/kg/i.p.) was administered and during the subsequent 15 minutes the frequency of the jumps was detected.

In three experiments (n = 24/group) the following results were obtained:

2

```
    ------------------------------------------

        Treatment               No. jumps

                                   X±S.E.

    ------------------------------------------

        Morphine                86.09±13.43

        Morphine+Dividol®       *42.13± 7.23

    ------------------------------------------

    * p<0.05 (Dunnet t)
```

## Claims

1. A process for the preparation of a pharmaceutical composition for the treatment of drug dependence containing Viminol.

2. A process according to claim 1 in which the pharmaceutical composition is in the form of tablets, capsules, coated tablets, suppositories, drinking solutions, injectable solutions or freezedried powders.

3. A process according to claim 1 or 2 in which the composition contains an amount of Viminol between 100 and 800 mg.